# EUROPEAN PATENT APPLICATION

(11) **EP 2 692 339 A2**
(43) Date of publication of application: **05.02.2014**
(21) Application number: 12726723.5
(22) Date of filing: 30.03.2012
(51) Int. Cl.: A61K 8/92, A61Q 19/10

(54) **COSMETIC CLEANSING COMPOSITION COMPRISING VEGETABLE OILS**

(30) Priority: 31.03.2011 BR PI1101136
(71) Applicant: Natura Cosmeticos S.A., 06882-700 ltapecerica da Serra - SP (BR)
(72) Inventor: BRASILINO DE CARVALHO, André Luís, São Bernardo do Campo SP (BR); PEREIRA LIMA, Rodrigo, Jundiaí SP (BR); RUBENS MIGUEL, Reinaldo, Jundiaí SP (BR); DE OLIVEIRA MARIN CHICOL, Tadeu, 07760-000 Cajamar SP (BR)
(74) Representative: Hewson, Timothy John
(86) International application number: PCT/BR2012/000087
(87) International publication number: WO 2012/129626

(57) **Abstract**

The present invention relates to a cosmetic composition, particularly indicated for skin cleaning, which comprises:
- at least one plant oil comprising 40% to 60% of fatty chain containing 12 carbons, C12, such chain may be saturated or unsaturated;
- at least one saponifying agent; and
- an aqueous portion.

The invention also relates to a process for preparing said cosmetic composition, as well as the use thereof.

## Description

### FIELD OF THE INVENTION

The present invention relates to cosmetic industry technologies. Particularly, the present invention relates to cosmetic compositions useful in personal hygiene and care, particularly in the form of soaps and toilet soaps comprising vegetable (plant) oils. The processes for making them are also described.

### BACKGROUND OF THE INVENTION

Soaps and toilet soaps are cosmetic products with marked detergent action, which are useful in personal hygiene of the body, face, hands and hair. Typically, they come in the form of a bar, paste, cream or liquid (VILLA, 2007).

The replacement of synthetic actives by natural actives in cosmetic compositions has been more and more sought, be it for the appeal of products of natural origin or for the improved chemical and cosmetic benefits which they provide (CHITWOOD, 2002).

In the prior referring to the present invention, one can see that plant oils find application in the field of cosmetics:
Patent application PCT/FR2003/00006 of Laboratoires Expanscience describes cosmetic compositions containing murumuru grains oil and a cosmetic treatment method which comprises administering said compositions.

Brazilian patent document PI0301420-7 relates to a formulation for murumuru toilet soap.

Brazilian patent document PI0106625-0 of Chemyunion Química Ltda. discloses the use of natural or purified and stable plant fat, extracted from the fruits of paims of the genus *Astrocaryum* in enhancing dermal and/or capillary moisturizing with respect to other usually employed plant fats, which can be used in hygiene products, cosmetics and pharmaceutical products.

Brazilian patent document PI0303405-4 of Chemyunion Química Ltda. describes the use of almond fat from the fruits of the palm of the genus *Astrocaryum,* as an additive for toilet soap, improving the skin barrier and enhancing the moisturizing power and general performance of toilet soaps prepared for normal, oily and sensitive skins.

Patent application PCT/BR2006/000193 relates to multifunctional cosmetic compositions in the form of w/o emulsions, which comprise a silicone system, an emollient system that may be composed of murumuru butter in an amount ranging from 1.0% to 5.0% by mass and cosmetically acceptable carriers.

At present, there are on the market of cosmetics products that use, as raw materials, oils extracted from the murumuru fruits, as for example:
- Chemysoap active (Chemyunion): a product that assembles saponified triglycerides from palms of the genus *Astrocaryum sp.,* employing them as additives in toilet soap formulations (SILVA, 2003; electronic site of Chemyunion)
- BR Forest butter (Chemyunion): natural butter from palms of the genus *Astrocaryum sp* (electronic site of Chemyunion)
- Muru-Muru 80g toilet soap - Organic (Folhata Cosméticos): toilet soap in bar, comprising murumuru (site of Folhata Cosméticos)
- Hair lotion Hidrataçäo Intensiva Murumuru Phytoervas (Grupo Nasha): a hair lotion comprising murumuru butter (site of the Grupo Nasha)

However, none of the prior art teachings presents solutions to the technical problems related to the workability of plant oils like murumuru butter, and to the organoleptic and physical characteristics of the final product. Such technical problems were solved according to the present invention.

### BRIEF DESCRIPTION OF THE INVENTION

The present invention relates to a cosmetic composition, particularly indicated for cleaning skin, which comprises:
- at least one plant oil comprising 40% to 60% of fatty chain containing 12 carbons, C12, such chain may be saturated or unsaturated;
- at least one saponifying agent; and
- an aqueous portion.

Particularly, said composition may further comprise one or more substances selected from the group consisting of active principles, di-phosphonates, wetting agents, thickening agents, chelating agents, sunscreens, dyes, antioxidants, perfumes, surfactants and water.

The invention further relates to a process for preparing a cosmetic composition, which involves improved workability of plant oils and imparts improved properties to the final product and which comprises the following steps:
a) preparing at least one plant oil comprising 40% to 60% of fatty chain containing 12 carbons, C12, such chain may be saturated or unsaturated:
b) heating the plant oil of step a) up to a temperature of about 90° to 95°C;
c) after heating, adding slowly at least one saponifying agent, under constant stirring and at a high rotation;
d) adding slowly an aqueous portion;
e) checking the reduction of vapor formed with the reaction.

It is also an objective of the invention the use of said cosmetic compositions.

### DETAILED DESCRIPTION OF THE INVENTION

The present invention relates to a cosmetic composition, particularly indicated for skin cleaning, which comprises:
- at least one plant oil comprising 40% to 60% of fatty chain containing 12 carbons, C12, such chain may be saturated or unsaturated;
- at least one saponifying agent; and
- an aqueous portion.

Preferably, the cosmetic composition of the present invention comprises:
- at least one plant oil comprising 40% to 60% of fatty chain containing 12 carbon atoms, C12, such chain may be saturated or unsaturated, in an amount ranging from 50% to 90% by mass;
- at least one saponifying agent in an amount ranging from 15% to 20% by mass; and
- water.

All the amounts are based on the total mass of the cosmetic composition.

In a preferred embodiment, the cosmetic composition of the present invention comprises:
- murumuru butter in an amount ranging from 50% to 90% by mass;
- sodium hydroxide in an amount ranging from 15% to 20% by mass; and
- water.

All the amounts are based on the total mass of the cosmetic composition.

The main examples of cosmetic products that can be prepared starting from the cosmetic composition of the present invention are:
❖ toilet soap bar;
❖ toilet soap chip;
❖ exfoliating toilet soap;
❖ pelletized toilet soap.

### Plant oil

The cosmetic composition of the present invention comprises at least one plant oil comprising 40% to 60% of fatty chain having 12 carbons, C12, such chain may be saturated or unsaturated, preferably in an amount ranging from 50% to 90% by mass.

Plant oils that meet the above requirement may be selected from: murumuru butter, babassu oil, palmist oil, inajá oil, tucuma oil, pupunha oil. Other plant oils comprising 40% to 60% of fatty chain containing 12 carbons, C12, such chain being saturated or unsaturated, may also be used.

Preferably, one employs murumuru butter in the cosmetic composition of the present invention.

Murumuru (scientific name *Astrocaryum murumuru* Mart.) is a variety of palm very common in the flooded regions of Amazonia (ROCHA & POTIGUARA, 2007). Its fruit contains about three times as much beta-carotene as carrot and its oil presents the following components, among others: *Astrocaryum murumuru* acid (or its astrocaryum murumuruate salt), lauric acid, myristic acid, oleic acid, palmitic acid (or its palmate or palm kernellate salt), capric acid, linoleic acid, stearic acid (or its stearate salt), caprilic acid and distyrylbiphenyl disulfonic acid (or its disulfonate salt). The fruit further contains proteins, lipids, vitamin A, calcium, phosphorus, iron, thiamine, riboflavin, niacine and ascorbic acid (ARAUJO ET AL, 2005; SILVA ET AL, 2008).

*Astrocaryum murumuru* acid (or its astrocaryum murumuruate salt) corresponds to saponified triglycerides characteristic of murumuru, having surfactant, tranquilizing and emollient functions (PEREIRA, 2007).

Palmitic acid (or its palmate or palm kernellate salt) corresponds to a fatty acid with surfactant, emulsifying and viscosity-controlling functions (THE ENVIRONMENTAL WORKING GROUP, 2010, a).

Myristic acid is insoluble in water and soluble in ethanol. It is used in cosmetology as a possible substitute for stearic acid, but chiefly esterified with isopropyl alcohol, giving origin to an oil very appreciated for its degree of penetration and stability (isopropyl myristate) (ARAÚJO ET AL, 2005).

Stearic acid (or its stearate salt) corresponds to a fatty acid with surfactant and emulsifying functions (PEREIRA, 2007).

Lauric acid behaves as a carrier of active principles, since it is capable of increasing their permeability through the skin. Its performance takes place in two different ways: by reaction with cationic actives, increasing their lipofilicity, or by temporary disorganization of the corneum layer of the skin. After undergoing neutralization reaction in the presence of a strong base, such as sodium hydroxide, lauric acid behaves as an emulsifier, stabilizing emulsion of the oil-in-water type (o/w) (ARAÚJO ET AL, 2005).

The distyrylbiphenyl disulfonic acid (or its disulfonate salt) is an organic substance with surfactant, viscosity-controlling and UV radiation absorbing (sunscreen) functions (THE ENVIRONMENTAL WORKING GROUP, 2010, b).

Oleic acid can be used as a chemical absorption promoter, since it is capable of improving the diffusion of active principles by the stratum corneum. This property is explained by its capability of modifying, in a reversible manner, the resistance thereof or by the reaction between this acid and cationic actives, generating salts with higher lipophilic nature (ARAÚJO ET AL, 2005).

In a first preferred embodiment of the present invention, the cosmetic composition comprises murumuru butter in an amount ranging from 50% to 90% by mass, the amounts being based on the total mass of the composition.

In a second preferred embodiment of the invention, the cosmetic composition comprises murumuru butter combined with palm oil in an amount ranging from 50% to 90% by mass, wherein the mixture should comprise from 40% to 60% of fatty chain containing 12 carbons.

### Saponifying agent

Saponification is basically the interaction which takes place between a fatty acid existing in oils with a strong base under heating. The soap is a carboxylic acid salt and, for having a long carbonic chain in its molecular structure, it is capable of solubilizing in both polar and non-polar medium.

For preparing the cosmetic composition of the present invention one uses a saponifying agent, which is preferably a strong base.

Preferably, it is adds, as saponifying agent, sodium hydroxide in an amount ranging from 15% to 20% by mass.

### Optional components

In order to impart to the cosmetic composition of the present invention some desirable characteristic which has not yet been achieved with the components cited, one may add optional components that are compatible with the properties thereof. A few of these components that may be added to the composition are those described hereinafter:
❖ Diphosphonates (or bisphosphonates) may be present in amounts ranging from about 0.1 to about 0.20% by mass and be selected from the group consisting of ethidronic acid, Turpinal SL and a mixture thereof;
❖ Humectants (or moisturizers or emollients) may be present in amounts ranging from about 1 to about 8.00% by mass and be selected from the group consisting of propyleneglycol, sucrose, sorbitol, glycerin, vaseline, mineral oil (s) and a mixture thereof;
❖ Hardness agents may be present in amounts ranging from about 0.10% to about 0.50% by mass and be selected from the group consisting of sodium chloride, sodic carboxymethylcellulose, di-etanolamine of fatty acid (s) and a mixture thereof;
❖ Chelating agents may be present in amounts ranging from 0.30% to about 0.70% by mass and be selected from the group consisting of tetrasodic EDTA;
❖ Dyes (or pigments) may be present in amounts ranging from 0.001% to about 0.030% by mass;
❖ Antioxidant agents may be present in amounts ranging from about 0.01% to about 0.10% by mass and be selected from the group consisting of BHT (butyl-hydroxytoluene);
❖ Surfactants may be present in amounts ranging from about 0.10% to about 5.00% by mass and be selected from the group consisting of decyl glycoside, lauryl sodium sulfate and a mixture thereof;
❖ Perfume (or essence) may be present in amounts ranging from about 0.01 % to about 2.00% by mass;
❖ Film former for toilet soap in bar, preferably lecithin, in an amount ranging from about 0.10% to 1.00% by mass;
❖ Other cosmetically acceptable components.

All the amounts presented for the optional components are based on the total mass of the final formulation of a toilet soap containing the composition of the present invention. Preferably, the composition of the present invention should be present in amounts ranging from 70 to 95% by mass, based on the total mass of the final formulation of a toilet soap.

The present invention provides, in one of the preferred embodiments, a toilet soap of uniform specific consistency, using a simple, efficient and cost-saving manufacture process.

The cosmetic composition of the present invention presents a range of advantages and characteristcs desired in a cosmetic product for the skin, these advantages being achieve with the optimum combination between the components already described, some of which are listed below:
❖ It provides efficient cleaning of the skin, making it smooth, silky, soft;
❖ It provides high foaming;
❖ The cosmetic composition does not dry as time passes;
❖ The cosmetic composition used in form of toilet bar soap or toilet chip soap does not have a brittle appearance;
❖ The cosmetic composition of the present invention is malleable, which enables manufacture of toilet soaps in different shapes, for example in the form of chips;
❖ The cosmetic composition of the present invention brings a great innovation in the socio-environmental field: the incorporation of 20% to 50% of oils from the Brazilian Biodiversity in manufacturing the vegetable masses. This work has generated jobs from over 830 associates of 10 new cooperatives/communities in the surroundings of the Saboaria Natura in the city of Benevides, PA, Brazil.

### Examples of toilet soap formulation according to the present invention

### Formulation Example 1

| Component | Concentration (% by weight) |
|---|---|
| Etidronic acid | 0.1000 |
| Demineralized water | 1.3000 |
| Sodium chloride | 0.5000 |
| Titanium dioxide | 0.3000 |
| Tetrasodic EDTA | 0.5000 |
| Propylene glycol | 1.3000 |
| Sorbitol | 5.0000 |
| Murumuru butter | 0.1000 |
| BHT | 0.0500 |
| Lauryl sodium sulfate | 1.7000 |
| Mass for toilet soap | 87.4500 |
| Mint murumuru fig | 1.7000 |

### Formulation Example 2

| Component | Concentration (% by weight) |
|---|---|
| Etidronic acid | 0.1000 |
| Demineralized water | 1.3000 |
| Sodium chloride | 0.5000 |
| Titanium dioxide | 0.3000 |
| Tefrasodic EDTA | 0.5000 |
| Propylene glycol | 1.3000 |
| Sorbitol | 5.0000 |
| BHT | 0.0500 |
| Lauryl sodium sulfate | 1.7000 |
| Mass for toilet soap | 87.5500 |
| Mint murumuru fig | 1.7000 |

### Formulation Example 3

| Component | Concentration (% by weight) |
|---|---|
| Etidronic acid | 0.1000 |
| Demineralized water | 0.5000 |
| Ultramarines | 0.0275 |
| Tetrasodic EDTA | 0.5000 |
| Propylene glycol | 1.3000 |
| Sorbitol | 5.0000 |
| Murumuru butter | 0.1000 |
| BHT | 0.0500 |
| Lauryl sodium sulfate | 1.7000 |
| Yellow iron oxide | 0.0500 |
| C1 black iron oxide | 0.0050 |
| Mass for toilet soap | 88.9675 |
| Mint murumuru fig | 1.7000 |

### Formulation Example 4

| Component | Concentration (% by weight) |
|---|---|
| Etidronic acid | 0.1000 |
| Demineralized water | 0.5000 |
| Ultramarines | 0.0275 |
| Tetrasodic EDTA | 0.5000 |
| Propylene glycol | 1.3000 |
| Sorbitol | 5.0000 |
| BHT | 0.0500 |
| Lauryl sodium sulfate | 1.7000 |
| Yellow iron oxide | 0.0500 |
| C1 black iron oxide | 0.0050 |
| Mass for toilet soap | 89.0675 |
| Mint murumuru Fig | 1.7000 |

### Process of preparing the cosmetic composition of the present invention

The procedure for preparing the cosmetic composition of the present invention is superior to the usual procedures in that, starting from few components and simple steps, it provides the cosmetic composition of the present invention, which exhibits superior properties as compared with the products existing on the market.

The process of preparing the cosmetic composition of the present invention comprises the following steps:
a) Preparing at least one plant oil comprising 40% to 60% of fatty chain containing 12 carbons, C12, such chain may be saturated or unsaturated;
b) Heating the plant oil of step a) up to a temperature of about 90° to 95°C;
c) After the heating, adding slowly at least one saponifying agent, under constant stirring and at high rotation;
d) Adding slowly an aqueous portion;
e) Checking the reduction of vapor formed with the reaction.

Further, optional steps may be included in the process described above:
f) Adding to the mixture of phase e) etidronic acid in an amount ranging from 0.01 to about 0.20%;
g) Adding an electrolyte, preferably sodium chloride in an amount varying up to 0.5%, so as to provide adjustment of the hardness of the cosmetic composition, which will later have the form of a billet;
h) Adding to the mixture of step g) at least one surfactant so as to impart to the cosmetic composition greater foaming capability in an amount ranging from 0.10% to about 2.00%;
i) Adding to the mixture of step h) propylene and sorbitol in an amount ranging from 1 to 8% by mass, to improve the malleability of the cosmetic composition;
j) Adding to the mixture of step i) a chelating agent, being preferably EDTA in an amount ranging from 0.01 to about 0.20%.

All the amounts presented for the components of the optional steps are based on the total mass of the final formulation of a toilet soap that contains the composition of the present invention. Preferably, the composition of the present invention should be present in amounts ranging from 70 to 95% by mass, based on the total mass of the final formulation of a toilet soap.

The process of preparing the cosmetic composition of the present invention results, then, in a cosmetic composition intended for skin cleaning, with a specific consistency.

The cosmetic composition resulting from the process detailed above, when in form of a billet, can be cut so as to form toilet bar soap or still be subjected to a specific tool to form soap chip.

The cosmetic composition of the present invention exhibits ideal malleability, hardness and emollience so that:
❖ It can be marketed in the form of toilet bar soaps, remaining sufficiently firm during its use or
❖ It can be sold in the form of chips obtained with:
   ○ Artisanal tools like knives capable of providing toilet soap chips or
   o Specific tools that can be incorporated into the process of manufacturing the toilet soap, at the end of the preparation of the billet, so that said billet can be subjected to simultaneous cuts, forming chips in series.

### CITED REFERENCES:

1) Araújo, V. F.; Petry, A.C.; Echeverria, R. M.; Fernandes, E. C.; Pastore Jr., F. "Plantas da Amazônia para Produção Cosmética: uma abordagem química - 60 espécies do extrativismo florestal não-madeireiro da Amazonia". Universidade de Brasilia - UnB, p. 18-20, 2005.
2) Chitwood, S. "Cosmética Natural", Ed. Aquariana, 5a Ed., 2002;
3) Environmental Working Group, a. Site available in http://www.cosmeticsdatabase.com/ingredient.php?ingred06=704412; accessed on May 12, 2010;
4) Environmental Working Group, a. Site available in http://www.cosmeticsdatabase.com/ingredient/702144/DISODIUM DI STYRYLBIPHENYL DISULFONATE/; accessed on May 12, 2010;
5) Pereira, C. M. "Tecnologia de Sabonetes", Centro Universitário das Faculdades Metropolitanas Unidas, 2007;
6) Rocha, C. B. R.; Potiguara, R. C. V. "Morfometria das fibras das folhas de Astrocaryum murumuru var. murumuru Mart. (Arecaceae)" Acta \\
7) Silva, C. R. "Sabonetes biomiméticos com ativos da Amazônia" Cosmetics & Toiletries, 5(15): 66-71, 2003;
8) Silva, E. P. O. S.; Castro, L.H.; Biaggio, R. M.; Beltrame Jr., M. "estudo das caracteristicas físico-químicas e classificação de fitoingredientes na espécie Astrocaryum murumuru (murumuru)" XII Encontro Latino-Americano de Iniciação Cientifica e VIII Encontro Latino-Americano de Pós-Graduação - Universidade do Vale do Paraíba, p. 1-3, 2008;
9) Villa, A. L. V. "Sabão e Sabonete" In: Apresentação de Cosmetologia do curso de Farmácia da Universidade Estácio de Sá - UNESA. 2007.

## Claims

1. A cosmetic composition **characterized by** comprising:
- at least one plant oil comprising 40% to 60% of fatty chain containing 12 carbons, C12, such chain may be saturated or unsaturated;
- at least one saponifying agent; and
- an aqueous portion.

2. A cosmetic composition according to claim 1, **characterized in that** the plant oil is present in an amount ranging from 50% to 90% by mass, based on the total mass of the composition.

3. A cosmetic composition according to claim 1 or 2, **characterized in that** the plant oil is selected from murumuru butter, babassu oil, palmist oil, inajá oil, tucuma oil, pupunha oil and mixtures thereof.

4. A cosmetic composition according to any one of claims 1 to 3, **characterized in that** the plant oil is murumuru butter.

5. A cosmetic composition according to any one of claims 1 to 3, **characterized by** comprising murumuru butter and palm oil.

6. A cosmetic composition according to any one of claims 1 to 5, **characterized in that** the saponifying agent is present in an amount ranging from 15% to 20% by mass, based on the total mass of the composition.

7. A cosmetic composition according to any one of claims 1 to 6, **characterized in that** the saponifying agent is sodium hydroxide.

8. A cosmetic composition according to any one of claims 1 to 7, **characterized in that** it is a toilet soap bar.

9. A cosmetic composition according to any one of claims 1 to 7, **characterized in that** it is a toilet soap chip.

10. A process for preparing a cosmetic composition, **characterized by** comprising the following steps:
- a) preparing at least one plant oil that comprises 40% to 60% of fatty chain containing 12 carbons, C12, such chain may be saturated or unsaturated;
- b) heating the plant oil of step a) up to a temperature of about 90° to 95°C;
- c) after heating, adding slowly at least one saponifying agent, under constant stirring and at high rotation;
- d) adding slowly an aqueous portion;
- e) checking the reduction of vapor formed with the reaction.

11. A process according to claim 10, **characterized in that** in step a) one uses murumuru butter.

12. A process according to claim 10, **characterized in that** in step a) one uses murumuru butter and palm oil.

13. A process according to any one of claims 10 to 12, **characterized in that** in step c) one uses sodium hydroxide.

14. Use of a cosmetic composition as defined in any one of claims 1 to 9, **characterized by** being in the preparation of a toilet soap for skin cleaning and hygiene.

15. Use of a cosmetic composition as defined in any one of claims 1 to 9, **characterized by** being in the preparation of a toilet soap for skin cleaning and hygiene.
